# EUROPÄISCHE PATENTANMELDUNG

(11) **EP 2 260 884 A1**
(43) Veröffentlichungstag der Anmeldung: **15.12.2010**
(21) Anmeldenummer: 09162307.4
(22) Anmeldetag: 09.06.2009
(51) Int. Cl.: A61L 31/02, A61L 31/14, A61N 1/00

(54) **Implantatsystem mit einem temporären Implantat und Verfahren zum Beeinflussung der Korrosionsrate eines Implantates**

(71) Anmelder: Heller, Jorg, 51766 Engelskirchen (DE)
(72) Erfinder: Heller, Jörg. Dr., D-51766 Engelskirchen (DE); Sieber, Irina. Dr., D-85051 Ingolstadt (DE); Oberhauser, SImon, D-85296 Rohrbach (DE)
(74) Vertreter: Bergmeier, Werner

(57) **Zusammenfassung**

Ein Implantatsystem (1) mit einem temporären Implantat (2) aus einem metallischen, biokompatiblen und biokorrodierbaren Werkstoff ist als elektrochemische Zelle ausgebildet. Das Implantat (2) bildet in vivo eine Arbeitselektrode (2), welche mit einer Gegenelektrode (3) elektrisch leitend und über Körpergewebe (5) des Patienten als Elektrolyt verbindbar ist. Das Implantat (2) ist hierdurch stabilisierbar oder auflösbar.

Bei einem Verfahren zur Beeinflussung der Korrosionsrate von temporären Implantaten (2) wird das Implantat (2) als eine erste Arbeitselektrode (2) ausgebildet und dem Patienten implantiert. Die Arbeitselektrode (2) wird elektrisch leitend sowie über Körpergewebe (5) des Patienten als Elektrolyt mit einer Gegenelektrode (3) verbunden, wobei eine elektrochemische Zelle gebildet und die Korrosionsrate des Implantats (2) in vivo gesteuert wird.

## Beschreibung

Die vorliegende Erfindung betrifft ein Implantatsystem mit einem temporären Implantat aus einem metallischen, biokompatiblen und biokorrodierbaren Werkstoff. Weiterhin betrifft die Erfindung ein Verfahren zur Beeinflussung der Korrosionsrate von temporären Implantaten aus einem metallischen biokorrodierbaren und biokompatiblen Werkstoff.

Medizinische Implantate sind in verschiedenen Ausgestaltungen aus dem Stand der Technik bekannt. Zu unterscheiden sind hierbei permanente Implantate, welche zum dauerhaften Verbleib im menschlichen Körper bestimmt sind und temporäre Implantate, welche nur zum zeitweisen Verbleib im Körper ausgebildet sind. Diese Implantate müssen, nachdem sie ihren Zweck erfüllt haben, in der Regel durch einen zweiten operativen Eingriff wieder aus dem Körper entfernt werden, da es zu unerwünschten Reaktionen des Körpers auf dem Fremdkörper kommen könnte. Als temporäre medizinische Implantate sind beispielsweise chirurgische bzw. orthopädische Schrauben oder Platten, Gefäßprothesen, Stents sowie Wirkstoffdepots und weitere bekannt.

Um die aufwendige operative Entfernung von Implantaten zu vermeiden, ist es bereits bekannt geworden, Implantate aus biokorrodierbaren bzw. biodegradierbaren Werkstoffen herzustellen. Bekannt geworden sind biodegradierbare Implantate aus Kunststoffmaterialien wie beispielsweise in der DE 2502884 C2 beschrieben, welche in erster Linie für Stents verwendet werden.

Metallische Werkstoffe sind zwar prinzipiell biokorrodierbar, es bestehen jedoch erhebliche Unterschiede in den Korrosionsgeschwindigkeiten. Als temporäre Implantate sind Stents aus Magnesium-Eisen-Legierungen bekannt. Diese korrodieren in vivo mit derart hohen Korrosionsraten, dass eine Explantation nicht erforderlich ist.. Insbesondere werden Magnesiumlegierungen als Implantate verwendet, da diese in vivo schnell korrodieren und nur geringe Toxizität aufweisen. Die Korrosionsraten von Magnesium in vivo sind jedoch vergleichsweise hoch, so dass die Implantate oftmals zu schnell korrodieren.

Daher ist es bereits bekannt geworden, auf Magnesiumimplantate eine Korrosionsschutzschicht aufzubringen, um die Korrosionsrate des Implantates in vivo zu beeinflussen. Die DE 10 2006 038 231 A1 schlägt beispielsweise vor, das Implantat mit einer Beschichtung aus einer Organo-Silizium-Verbindung zu überziehen, welche die Korrosionsrate verzögert, so dass die Implantate auch länger beständig bleiben. Die DE 10 2006 060 501 A1 schlägt hingegen vor, ein Magnesiumimplantat mit einer wässrigen oder alkoholischen Konversionslösung zu behandeln, um eine korrosionshemmende Schicht zu erzielen.

Eine weitere Möglichkeit, die Korrosionsgeschwindigkeit des Implantates in vivo zu beeinflussen, besteht in dem Zusatz bestimmter Legierungselemente sowie der Abstimmung der Materialstärke des Werkstoffes. Dies wird beispielsweise in der EP 1 270 023 B1 beschrieben.

Eine Beeinflussung des Korrosionsverhaltens von Implantaten ist somit nur über die Wahl der Werkstoffe und ihrer Legierungsbestandteile oder durch das Aufbringen bestimmter Beschichtungen möglich. Bei schnell korrodierenden Materialen wie Magnesium wird häufig trotz aufgebrachter Beschichtungen das Implantat vergleichsweise schnell aufgelöst, so dass ein Implantat auf Magnesiumbasis nur in bestimmten Fällen einsetzbar ist. Materialien wie Stahl weisen gegenüber Magnesiumwerkstoffen oftmals günstigere mechanische Eigenschaften auf, korrodieren jedoch in vivo zu langsam, so dass eine erneute Operation zur Entfernung des Implantates erforderlich ist. Aufgabe der vorliegenden Erfindung ist es daher, ein Implantatsystem und ein Verfahren vorzuschlagen, welche eine gezielte Beeinflussung des Korrosionsverhaltens eines Implantates in vivo ermöglichen.

Die Aufgabe wird gelöst mit den Merkmalen der unabhängigen Ansprüche.

Ein Implantatsystem mit einem temporären Implantat aus einem metallischen biokorrodierbaren Werkstoff ist erfindungsgemäß als elektrochemische Zelle ausgebildet. Das Implantat bildet hierbei in vivo eine Arbeitselektrode und ist mit einer Gegenelektrode leitend verbindbar. Das Körpergewebe des Patienten stellt den Elektrolyten der elektrochemischen Zelle dar. Im einfachsten Fall bildet die elektrochemische Zelle ein Korrosionselement. Wird das Implantat mit einer Opfer-Anode elektrisch leitend verbunden und ist an den Elektroden Körpergewebe des Patienten als Elektrolyt vorhanden, so wird die Opfer-Anode korrodiert und das kathodische Implantat stabilisiert. Ebenso kann jedoch das Implantat auch die Anode bilden und mit einer inerten Kathode verbunden werden, um eine Auflösung des Implantats herbeizuführen.

Durch die vorgeschlagene Anordnung ist es somit möglich, die Korrosionsrate des Implantates in vivo gezielt zu beeinflussen. Mit dem erfindungsgemäßen Verfahren bzw. dem erfindungsgemäßen Implantatsystem ist es durch die Ausbildung als elektrochemische Zelle möglich, Implantate bestehend aus einem Werkstoff mit einer hohen Korrosionsrate wie Magnesium gezielt zu stabilisieren, um die Verweildauer im Körper zu erhöhen. Ebenso kann mit dem erfindungsgemäßen Verfahren die Korrosionsgeschwindigkeit vergleichsweise langsam korrodierender Implantatwerkstoffe, beispielsweise Eisenwerkstoffen, gezielt erhöht werden. Das Implantat kann hierbei beliebige, einsatzangepasste Eigenschaften aufweisen. Die Erfindung ist somit in Verbindung mit den verschiedensten Implantaten und deren Einsatzzwecken anwendbar.

Besonders vorteilhaft ist es, wenn das Implantat und die Gegenelektrode mit einer Spannungs- oder Stromquelle verbindbar sind. Indem das Implantatsystem mit einer Spannung oder einem Strom beaufschlagt wird, ist eine gezielte Steuerung der Korrosionsrate des Implantates möglich. Die Korrosionsrate kann hierbei je nach Höhe der Spannung bzw. der Stromstärke erheblich verringert oder erhöht werden. Zudem ist es möglich, die Korrosionsrate konstant einzustellen oder den Erfordernissen entsprechend anzupassen.

Nach einer ersten Ausführung der Erfindung werden hierbei die Gegenelektrode und/oder die Spannungs- oder Stromquelle zusammen mit dem Implantat implantiert. Je nach Lage des Implantats im Körper kann die Gegenelektrode derart positioniert werden, dass sie vergleichsweise einfach wieder explantierbar ist, während die Stromquelle lediglich unter die Haut implantiert werden muss. Aufwendige operative Eingriffe zum Entfernen des Implantates sind somit nicht erforderlich.

Die Gegenelektrode und ggf. eine Brücke zur leitenden Verbindung mit einer Referenzelektrode können jedoch zum gewünschten Zeitpunkt auch perkutan appliziert werden. Die leitende Verbindung des Implantates, welches die Arbeitselektrode bildet, mit einer externen Spannungs- oder Stromquelle kann ebenfalls perkutan erfolgen. Die Gegenelektrode bzw. die leitenden Verbindungen sind in diesem Fall als Nadeln ausgebildet, welche problemlos auch bis in tiefere Gewebeschichten eingebracht werden können. Im Rahmen dieser Erfindung werden diese Nadeln allgemein als Einstichelektroden bezeichnet, auch wenn sie nicht die eigentliche Elektrode bilden.

Nach einer anderen Ausführung der Erfindung umfasst das Implantatsystem weiterhin eine externe Referenzelektrode, welche über einen Elektrolyten und eine leitende Brücke mit dem Körpergewebe verbindbar ist. Mittels eines Drei-Elektroden-Systems zur Durchführung des erfindungsgemäßen Verfahrens ist eine genaue Einstellung der Verfahrensparameter möglich. Je nach Lage des Implantates ist hierbei auch die beschriebene vergleichsweise einfache perkutane Einbringung der Gegenelektrode und/oder der Brücke als Einstichelektrode möglich.

Bei einem Verfahren zur gezielten Auflösung oder Stabilisierung von temporären Implantaten kann somit vorteilhafterweise die Gegenelektrode und/oder eine Brücke der Referenzelektrode nach Implantation der Arbeitselektrode in das Körpergewebe eingebracht werden. Es ist somit auch möglich, beispielsweise bei langsam korrodierenden Materialien nach Beendigung der Behandlung eine gezielte und schnelle Auflösung des Implantates vorzunehmen. Ebenso ist es jedoch möglich, die Referenzelektrode ebenfalls direkt zu implantieren, um eine Verbindung mit dem Körpergewebe herzustellen.

Weiterhin ist es vorteilhaft, wenn das Implantat eine Steuereinheit umfasst, mittels welcher eine Stromstärke zwischen den Elektroden oder ein Potential der Arbeitselektrode regulierbar ist. Besonders vorteilhaft ist es hierbei, wenn die Steuereinheit einen Potentiostaten beinhaltet. Je nach Implantatmaterial und Einsatzzweck kann mittels der Steuereinheit eine anodische Polarisierung oder auch eine kathodische Polarisierung vorgenommen werden.

Bei einem Verfahren zur Beeinflussung der Korrosionsrate von Implantatmaterialien in vivo ist es je nach Material und Einsatzzweck weiterhin vorteilhaft, wenn die Polarisierung der Arbeitselektrode potentiostatisch reguliert wird. Beinhaltet die Steuereinheit des Implantatsystems einen Potentiostaten, so ist die Regulierung in besonders günstiger Weise möglich.

Ebenso kann es jedoch auch vorteilhaft sein, wenn die Polarisierung der Arbeitselektrode galvanostatisch reguliert wird. Der Aufbau des Implantatsystems kann hierdurch konstruktiv besonders einfach gehalten werden, und kann in günstiger Weise zur Langzeitpolarisierung verwendet werden, da das System dann auch voll implantierbar ausgebildet sein kann.

Bei einer implantierbaren bzw. implantierten Spannungs- oder Stromquelle ist es weiterhin vorteilhaft, wenn diese in vivo von den Elektroden trennbar ist. Das Schalten der Spannungs- oder Stromquelle kann beispielsweise von außen durch Funk-, Infrarot- oder Magnetschalter erfolgen und ermöglicht es ebenfalls, eine Spannung oder einen Strom erst nach einer gewissen Zeitspanne oder einem Verlauf folgend aufzubringen. So kann beispielsweise das Implantat je nach Erfordernissen, beispielsweise in Abhängigkeit eines Krankheits-/Genesungsbildes über eine bestimmte Zeitspanne unbeaufschlagt bleiben, um die Beständigkeit des Implantats über zu gewährleisten. Um dann nach Ablauf dieser ersten Behandlungsperiode die Auflösung des Implantates einzuleiten, kann schließlich die Spannungsquelle geschaltet und das Implantat beaufschlagt werden. Mittels einer derartigen schaltbaren Spannungsquelle ist es in einfacher Weise möglich, die Polarisierung der Arbeitselektrode kontrolliert und in Abhängigkeit bestimmter Parameter vorzunehmen.

Besonders vorteilhaft ist es weiterhin, wenn die Polarisierung der Arbeitselektrode in Abhängigkeit eines Korrosionszustandes des Implantates in vivo oder in Abhängigkeit eines Krankheits-/Genesungsbildes reguliert wird. So ist es beispielsweise möglich, den Zustand des Implantates oder eine nachwachsende oder sich neu bildende Gewebe- oder Gefäßstruktur mittels verschiedener bildgebender Verfahren zu kontrollieren und das Potential der Arbeitselektrode oder eine Stromstärke zwischen den Elektroden durch die Steuereinheit entsprechend zu regulieren.

Besonders vorteilhaft ist es weiterhin, wenn die Elektroden innerhalb des Körpergewebes mit biodegradierbaren Leitungen versehen sind. Beispielsweise können von tief im Körperinneren liegenden Implantaten biodegradierbare Leitungen durch das Körpergewebe bis unter die Haut reichen. Um dann zum gewünschten Zeitpunkt die Polarisierung vorzunehmen, können wiederum wie beschrieben leitende Verbindungen als Einstichelektroden in vergleichsweise einfacher Weise perkutan eingebracht werden, um mit der biodegradierbaren Leitung verbunden zu werden. Es ist somit möglich, das erfindungsgemäße Verfahren auch bei tiefliegenden Implantaten, welche perkutan nicht direkt erreichbar sind, einzusetzen. Da nach der Auflösung des Implantates auch die Leitungen degradieren, sind langfristig keine Abstoßungsprobleme oder anderen Reaktionen durch verbliebene Leitungen mehr zu erwarten. Die Beständigkeit der Leitungen sollte hierbei auf die Korrosionsrate des Implantates abgestimmt werden, um die Funktionalität des Implantatsystems zu gewährleisten.

Weitere Vorteile der Erfindung werden anhand der nachfolgend dargestellten Ausführungsbeispiele beschrieben. Es zeigen:
- **Figur 1**: Eine erste Ausführung der Erfindung mit einem vollständig im- plantierbaren Implantatsystem mit einer Stromquelle,
- **Figur 2**: eine weitere Ausführung der Erfindung mit einem vollständig implantierbaren Implantatsystem ohne Stromquelle,
- **Figur 3**: eine weitere Ausführungsform der Erfindung mit einer galvano- statischen Polarisierung des Implantates,
- **Figur 4**: eine weitere Ausführung eines Implantatsystems mit einer Re- ferenzelektrode, die sich außerhalb des Körpergewebe befin- det,
- **Figur 5**: eine weitere Ausführung eines Implantatsystems mit einer Re- ferenzelektrode, die sich innerhalb des Körpergewebes befin- det, sowie
- **Figur 6**: eine Ausführung der Erfindung für schwer zugängliche Implan- tate.

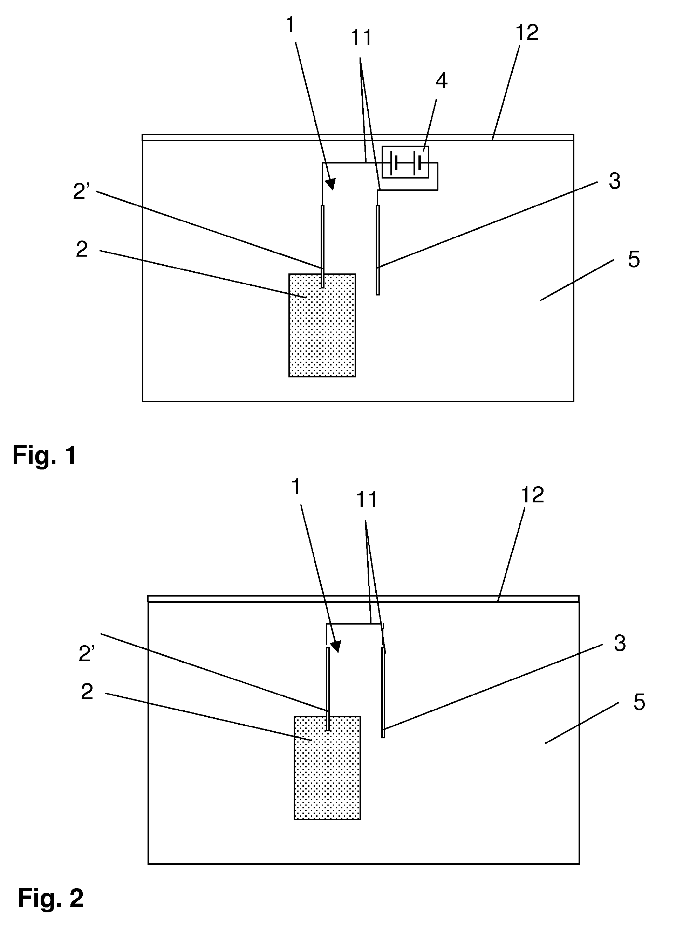

Figur 1 zeigt eine erste Ausführung der Erfindung mit einem vollständig implantierbaren Implantatsystem 1. Das Implantatsystem 1 umfasst hierbei das Implantat 2, welches die Arbeitselektrode 2 bildet, sowie als Gegenelektrode 3 eine inerte Elektrode, vorzugsweise aus Platin, Titan, Kobalt oder Grafit, welche ebenfalls in den Körper des Patienten implantiert ist. Weiterhin umfasst das Implantatsystem eine Stromquelle 4, welche im Körper des Patienten unter der Haut 12 implantiert ist. Das Körpergewebe 5 bildet in der vorliegenden Anordnung einen Elektrolyten, über welchen die Arbeitselektrode bzw. das Implantat 2 und die Gegenelektrode 3 leitend verbunden sind. Das Implantatsystem 1 bildet somit eine elektrochemische Zelle, durch welche bei gezieltem Anlegen eines Stromes die Korrosionsgeschwindigkeit des Implantates 2 in vivo gesteuert werden kann. Je nach Polarisierung der Arbeitselektrode 2 kann hierbei das Implantat 2 gezielt elektrochemisch aufgelöst werden oder aber auch, beispielsweise bei Implantaten 2, welche unter freiem Korrosionspotential zu schnell korrodieren, eine gezielte Stabilisierung des Implantates 2 vorgenommen werden.

Da nach der in Figur 1 gezeigten Ausführung der Erfindung nach Implantation des Implantatsystems 1 die Hautbarriere des Patienten wieder vollständig geschlossen ist, eignet sich das dargestellte Implantatsystem 1 auch zur Langzeitanwendung. So ist es beispielsweise möglich, ein Implantat 2 aus einem Magnesium-Werkstoff, welches unter freiem Korrosionspotential sehr schnell korrodiert, über einen längeren Zeitraum zu stabilisieren. Die Lebensdauer eines Implantates 2 auf Magnesiumbasis im Körper des Patienten kann hierdurch erheblich verlängert werden.

Magnesiumwerkstoffe werden unter freiem Korrosionspotential in Körpergewebe mit einer Korrosionsrate von bis zu 60 mm pro Jahr abgetragen. Hierbei entsteht eine starke lokale Korrosion (Lochfraßkorrosion), welche bereits nach kürzerer Zeit ein Implantat so schwächen kann, dass dieses seine Aufgabe nicht mehr erfüllen kann. Wird beispielsweise ein Magnesiumimplantat zur Fixierung von Knochenbrüchen eingesetzt, ist hierbei bedingt durch die starke lokale Korrosion eine Fixierung der Fraktur bereits nach vergleichsweise kurzer Zeit nicht mehr sicher gegeben.

Versuche des Anmelders zum Korrosionsverhalten von Magnesium, welche mit SBF (Simulated Body Fluids) als Elektrolyt zum Ersatz des Körpergewebes durchgeführt wurden, haben nun ergeben, dass durch gezielte kathodische Polarisierung eines Magnesiumimplantates die Abtragsraten erheblich reduziert werden können. So kann beispielsweise in entzündetem Körpergewebe (SBF mit pH 5,5) die jährliche Korrosionsrate um etwa den Faktor 4 gesenkt werden. Versuche des Anmelders haben gezeigt, dass der pH-Wert des Körpergewebes bzw. des SBF einen großen Einfluss auf die Korrosionsrate des Implantates hat. So wurden beispielsweise in normalem Körpergewebe bzw. SBF mit pH-Wert 7,4 unter freiem Korrosionspotential Korrosionsraten von etwa 10 mm pro Jahr ermittelt, kathodische Polarisierung des Implantates zeigt in diesem Fall keinen visuellen Korrosionsangriff. Weiterhin wurde ermittelt, dass die Zugabe von Albumin einen erheblichen Einfluss auf die Korrosionsrate hat. So konnten im Versuch mit SBF bei Albuminzugabe die Korrosionsraten etwa um das fünffache verringert werden.

Es ist somit durch gezielte kathodische Polarisierung eines Implantates 2 auf Magnesiumbasis möglich, die Korrosionsrate in Abhängigkeit von Parametern wie pH-Wert und Zugabe von Albumin und entsprechend der gewünschten Behandlungsdauer bzw. Einsatzdauer des Implantates 2 einzustellen. Die in Figur 1 dargestellte Anordnung kann jedoch ebenso auch bei einem Implantat 2 auf Stahlbasis eingesetzt werden, um die Korrosionsrate gezielt zu beschleunigen und eine gleichmäßige Degradation des Implantates 2 zu erzielen.

Nach Abschluss der Langzeitpolarisierung des Implantates 2 müssen nur noch die Gegenelektrode 3, die Stromquelle 4 sowie ggf. Leitungen 11 explantiert werden, was nur kleine Schnitte erfordert und im Vergleich zur Explantation des Implantates 2 nur eine geringe Belastung des Patienten darstellt. Je nach Ort und Formgebung des Implantates 2 kann hierbei die Gegenelektrode 3 auch nahe unter der Haut implantiert sein, so dass die Belastung des Patienten hierdurch noch weiter verringert ist.

Bei der dargestellten Anordnung ist es weiterhin möglich, die Stromquelle 4 schaltbar mit den Elektroden 2 und 3 zu verbinden. Wird die gezeigte Anordnung beispielsweise zur gezielten Auflösung eines Implantates 2 eingesetzt, so kann mittels einer schaltbaren Stromquelle 4 das Implantat 2 zunächst spannungslos gehalten werden, um die Funktion des Implantats nicht zu beeinträchtigen. Nach Ablauf einer bestimmten Zeitspanne kann durch Schalten der Stromquelle 4 die Polarisierung der Arbeitselektrode 2 vorgenommen werden. Eine Betätigung der Schalteinrichtung ist mittels Magnetschalter oder Funk denkbar. Mittels einer derartigen Anordnung ist es in gewissem Umfang möglich, den Zustand der Arbeitselektrode 2 bzw. den Behandlungsprozess mittels bildgebender Verfahren zu kontrollieren und die Polarisierung der Arbeitselektrode in Abhängigkeit dieser Parameter vorzunehmen.

Figur 2 zeigt eine Ausführung der Erfindung, bei welcher das Implantatsystem vollständig implantiert ist. Im Unterschied zur Ausführung der Figur 1 weist das Implantatsystem jedoch keine Stromquelle 4 auf. Diese vergleichsweise einfache Ausführung der Erfindung ist bereits ausreichend, um eine Stabilisierung oder Auflösung des Implantats herbeizuführen. Die Korrosionsrate des Implantats hängt hierbei nur vom Werkstoff des Implantats sowie dem Material der Gegenelektrode ab.

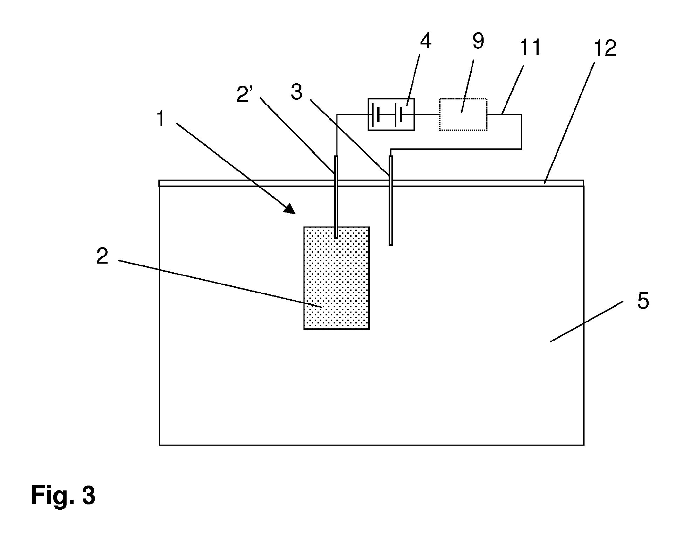

Figur 3 zeigt eine andere Ausführungsform der Erfindung, bei welcher lediglich das Implantat als Arbeitselektrode 2 fest implantiert ist. Die Stromquelle 4 ist außerhalb des Körpers angeordnet und eine leitende Verbindung zum Implantat 2 bzw. in das Körpergewebe 5 des Patienten erfolgt im vorliegenden Beispiel durch Einstichelektroden 2', 3. Die Einstichelektroden 2', 3 können in einfacher Weise perkutan appliziert werden, wobei es auch möglich ist, tief unter der Haut angeordnete Implantate 2 zu erreichen. Da hierbei die schützende Hautbarriere durchbrochen wird, besteht die Gefahr, dass Keime von den außenliegenden Teilen der Elektroden 2', 3 sich in das Körperinnere ausbreiten, so dass eine erhöhte Infektionsgefahr besteht. Die Anwendung mit einer externen Gleichstromstelle und Einstichelektroden 2', 3 ist somit nur zur Anwendung über einen vergleichsweise kurzen Zeitraum geeignet. Beispielsweise ist es möglich, Implantate 2 aus Eisenwerkstoffen durch eine anodische Polarisierung aufzulösen. Das Implantatsystem 1 weist weiterhin eine Steuereinheit 9 auf, mittels welcher die jeweils gewünschte Stromstärke einstellbar ist.

Versuche des Anmelders in SBF haben hierbei ergeben, dass die Korrosionsrate von Eisen auf bis zu 70 mm pro Jahr erhöht werden kann, was in etwa dem 70-fachen der freien Korrosionsrate entspricht. Auch hier wurden erhebliche Einflüsse des pH-Wertes sowie der Zugabe von Albumin auf die Korrosionsrate festgestellt. Insbesondere in entzündetem Körpergewebe (SBF mit pH-Wert 5,5) konnte ein erheblicher Einfluss der Albuminkonzentration festgestellt werden, welcher den Korrosionsangriff beschleunigte. Weiterhin wurde für entzündetes Körpergewebe ein eher gleichmäßiges Korrosionsbild ermittelt, während bei höherem pH-Werten eine eher lokale Korrosion auftrat.

Da die Korrosionsrate bei Eisenwerkstoffen auf bis zu 70 mm pro Jahr beschleunigt werden kann, ist es somit mit der in Figur 3 beschriebenen Anordnung möglich, nach Ende der Behandlung das Implantat 2 durch galvanostatische Anodisierung in vergleichsweise kurzer Zeit und bei gleichmäßigem Abtrag aufzulösen.

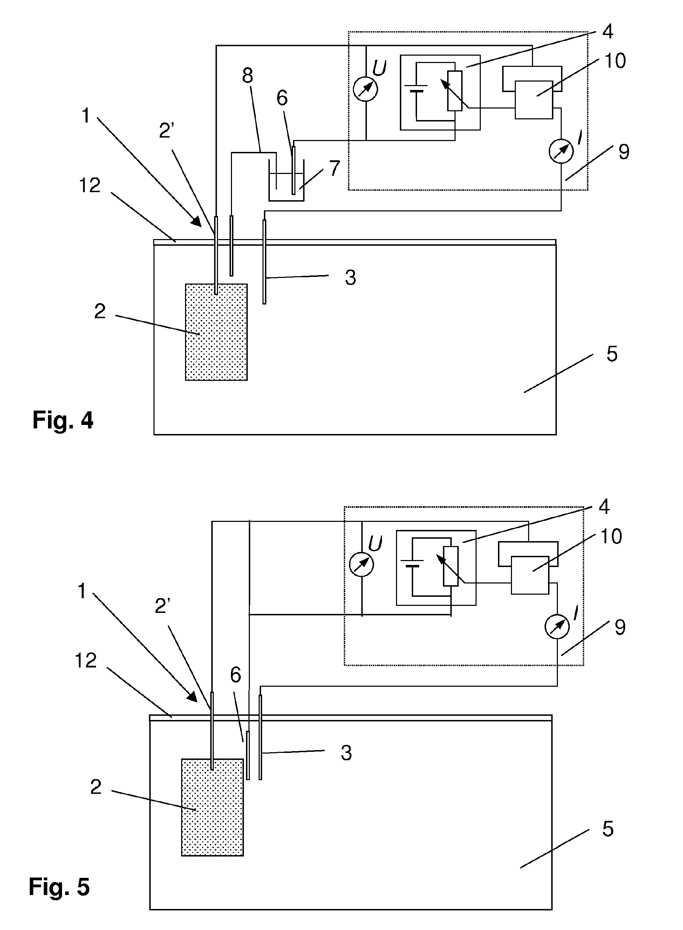

Figur 4 zeigt eine weitere Ausführung eines erfindungsgemäßen Implantatsystems 1 in einer Drei-Elektroden-Anordnung. Neben der Arbeitselektrode 2, der Gegenelektrode 3 und der Spannungs- oder Stromquelle 4 umfasst das Implantatsystem 1 weiterhin eine Referenzelektrode 6, welche ebenfalls über das Körpergewebe 5 leitend mit der Arbeitselektrode 2 in Verbindung steht. Die Verbindung der Referenzelektrode 6 mit dem Körpergewebe 2 erfolgt hierbei über einen Elektrolyten 7 und eine Brücke 8. Wie aus der Darstellung ersichtlich, sind hierbei die Brücke 8, die Gegenelektrode 3 sowie die Elektrode 2' bzw. die leitende Verbindung zum Implantat 2 als Einstichelektroden ausgebildet. Mittels der gezeigten Drei-Elektroden-Anordnung ist neben der bereits beschriebenen galvanostatischen Polarisierung auch eine potentiostatische Polarisierung der Arbeitselektrode 2 möglich. Beispielsweise hat sich bei Eisenwerkstoffen eine potentiostatische Polarisierung als sehr vorteilhaft erwiesen, um hohe Abtragsraten zu erzielen. Ebenfalls möglich ist eine potentiostatische Polarisierung außerhalb des Körpers, wie in Figur 3 dargestellt, zur Auflösung von Metallschäumen, z. B. aus Eisen, unlegierten Stählen und Wolfram.

Um die Polarisierung der Arbeitselektrode 2 gezielt durchführen zu können, ist es sowohl bei galvanostatischer Polarisierung, wie in den Figuren 1 und 2 beschrieben, wie auch bei potentiostatischer Polarisierung vorteilhaft, wenn das Implantatsystem 1 eine Steuereinheit 9 umfasst. Mittels der Steuereinheit 9 ist dann die Stromstärke zwischen den Elektroden 2, 3 bzw. das Potential der Arbeitselektrode 2 regulierbar. Nach der Darstellung der Figur 3 beinhaltet die Steuereinheit 9 einen Potentiostaten oder Galvanostaten 10, mittels welchem die Polarisierung der Arbeitselektrode 2 regulierbar ist. Hierbei wird in an sich bekannter Weise das Potential der Arbeitselektrode 2 gegen die Referenzelektrode 6 gemessen, während der Stromfluss über die Gegenelektrode 3 erfolgt. Vorzugsweise ist an der Spannungs- oder Stromquelle 4, wie hier dargestellt, eine Sollspannung einstellbar. Die Sollspannung kann beispielsweise je nach verwendetem Implantatmaterial, dem Zustand des Implantates 2 in vivo, dem Behandlungsfortschritt oder in Abhängigkeit weiterer Parameter eingestellt werden. Es ist jedoch auch möglich, eine Spannungs- oder Stromquelle 4 mit fester Spannung oder festem Strom einzusetzen.

Potentiostatische Untersuchungen des Anmelders zur Abtragung von Stahl haben beispielsweise bei einem Potential von 0 V gegenüber der Referenzelektrode 6 Abtragsraten von ca. 70 mm pro Jahr ergeben. Eine Beeinflussung der Abtragsrate ist hierbei durch die Einstellung des Potentials an der Arbeitselektrode 2 möglich, so dass die Lebensdauer des Implantates 2 in vivo vergleichsweise genau auf die Bedürfnisse abgestimmt werden kann. Für die Stabilisierung eines Magnesiumimplantates hat sich ein Potential von etwa -2 V gegenüber der Referenzelektrode 6 als vorteilhaft erwiesen. Als Referenzelektrode 6 ist beispielsweise eine gesättigte Kalomel-Elektrode einsetzbar.

Figur 5 zeigt im wesentlichen die Ausführung der Erfindung nach Figur 4, wobei jedoch die Referenzelektrode 6 sich ebenfalls innerhalb des Körpergewebes befindet. Eine Brücke sowie ein externer Elektrolyt sind hierbei nicht erforderlich, was die Handhabung des Implantatsystems erleichtert.

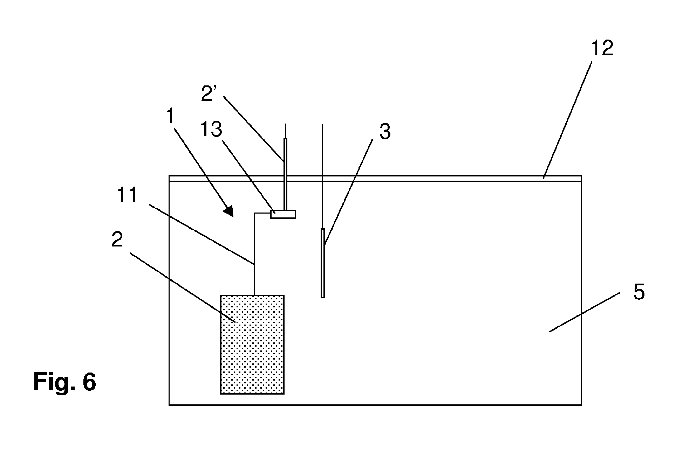

Figur 6 zeigt eine weitere Ausführungsform der Erfindung, welche insbesondere bei tief liegenden, schlecht erreichbaren Implantaten 2 einsetzbar ist. So kann beispielweise ein Implantat 2, welches mittels Einstichelektroden 2', 3 nur schlecht erreichbar ist, mit einer Leitung 11 versehen werden, welche ebenfalls implantierbar ist. Diese kann unterhalb der Haut 12 einen Anschluss 13 beinhalten. Um die leitende Verbindung einer externen Spannungs- oder Stromquelle 4 (hier nicht dargestellt) zu dem Implantat 2 herzustellen, kann dann ebenfalls eine Einstichelektrode 2' im Bereich der Leitung 11 bzw. des Anschlusses 13 eingebracht werden. Die Gegenelektrode 3 kann je nach den Gegebenheiten wie bereits beschrieben entweder als Einstichelektrode ausgebildet sein oder ebenfalls unter der Haut 12 implantiert sein.

Zum Entfernen der Elektrode 3 und ggf. des Anschlusses 13 sind dann jeweils wiederum nur kleine Schnitte erforderlich. Besonders vorteilhaft ist es hierbei, wenn wenigstens die Leitung 11 und ggf. auch der Anschluss 13 ebenfalls aus einem biodegradierbaren Material hergestellt sind. Diese müssen somit wie das Implantat 2 nicht entfernt werden, so dass auch hierdurch die Belastungen des Patienten weiter reduziert werden können. Die Korrosionsrate der biodegradierbaren Leitungen 11 sowie des Anschlusses 13 ist hierbei auf die Korrosionsrate des Implantates 2 abzustimmen, so dass eine leitende Verbindung für die Polarisierung gewährleistet ist. Dies ist ggf. durch Abstimmung der Materialien sowie bestimmter Beschichtungen möglich. Ebenso ist es jedoch auch möglich, die Leitung 11 sowie ggf. den Anschluss 13 nach Beendigung der Behandlung wieder zu explantieren. Auch dies ist im Vergleich zur Explantation eines herkömmlichen und vergleichsweise großen Implantates 2 in vergleichsweise einfacher Weise und ohne große Belastungen des Patienten möglich.

Um eine leitende Verbindung des Implantates 2 und/oder der Elektrode 3 durch die Haut 12 nach außen zu erzielen, ist es weiterhin auch möglich, eine Leitung 11 durch die Haut 12 nach außen zu legen. Da auch hier die Gefahr des Eindringens von Keimen in das Körperinnere besteht, ist ein derartiges System jedoch nicht zur Langzeitpolarisierung geeignet. Die Entfernung der Leitungen und ggf. der Elektrode 3 stellt auch hier im Vergleich zur Explantation des Implantats 2 nur eine geringe Belastung für den Patienten dar.

Die Erfindung ist nicht auf die dargestellten Ausführungsbeispiele beschränkt. So ist beispielsweise anstelle von Eisenwerkstoffen und Magnesium eine Vielzahl von weiteren Implantatwerkstoffen möglich, welche gezielt in ihrem Korrosionsverhalten beeinflusst werden können. Weiterhin sind als Implantate nicht nur Knochenimplantate, sondern verschiedenste temporäre Implantate möglich. So ist es beispielsweise auch möglich, Stents aus einem Magnesiummaterial zu fertigen und durch die kathodische Polarisierung zu stabilisieren. Die Korrosionsgeschwindigkeit kann hierbei vorteilhafterweise derart eingestellt werden, dass je nach Einsatzzweck des Stents und weiteren Gegebenheiten eine vollständige Degradation bereits nach wenigen Wochen erfolgt. Ist es erforderlich, dass Gefäßlumen über einen längeren Zeitraum abzustützen, so kann das Implantat auch über einen Zeitraum von beispielsweise sechs Monaten bis zu einem Jahr stabilisiert werden. Weiterhin ist die Erfindung für die verschiedensten temporären chirurgischen oder orthopädischen Implantate einsetzbar. Weitere Abwandlungen und Kombinationen im Rahmen der Patentansprüche fallen ebenfalls unter die Erfindung.

### Bezuaszeichenliste

- 1: Implantatsystem
- 2: Implantat, Arbeitselektrode
- 3: Gegenelektrode
- 4: Spannungs- oder Stromquelle
- 5: Körpergewebe
- 6: Referenzelektrode
- 7: Elektrolyt
- 8: Brücke
- 9: Steuereinheit
- 10: Potentiostat, Galvanostat
- 11: Leitung
- 12: Haut
- 13: Anschluss

## Patentansprüche

1. Implantatsystem (1) mit einem temporären Implantat (2) aus einem metallischen, biokompatiblen und biokorrodierbaren Werkstoff, **dadurch gekennzeichnet, dass** das Implantatsystem (1) als elektrochemische Zelle ausgebildet ist, wobei das Implantat (2) in vivo eine Arbeitselektrode (2) bildet, welche mit einer Gegenelektrode (3) elektrisch leitend und über Körpergewebe (5) des Patienten als Elektrolyt verbindbar ist, und das Implantat (2) hierdurch stabilisierbar oder auflösbar ist.

2. Implantatsystem nach dem vorherigen Anspruch, **dadurch gekennzeichnet, dass** das Implantat (2) und die Gegenelektrode (3) mit einer Spannungs- oder Stromquelle (4) verbindbar sind.

3. Implantatsystem nach dem vorherigen Anspruch, **dadurch gekennzeichnet, dass** die Gegenelektrode (3) eine inerte Elektrode aus einem biokompatiblen Werkstoff ist.

4. Implantatsystem nach dem vorherigen Anspruch, **dadurch gekennzeichnet, dass** die Gegenelektrode (3) und/oder die Spannungs- oder Stromquelle (4) implantierbar ausgebildet sind.

5. Implantatsystem nach einem der vorherigen Ansprüche, **dadurch gekennzeichnet, dass** das Implantatsystem (1) eine externe Referenzelektrode (6) umfasst, welche über eine Brücke (8) mit dem Körpergewebe (5) verbindbar ist oder direkt implantierbar ist.

6. Implantatsystem nach einem der vorherigen Ansprüche, **dadurch gekennzeichnet, dass** wenigstens eine der Elektroden (2', 3) und/oder die Brücke (8) perkutan applizierbar ist.

7. Implantatsystem nach einem der vorherigen Ansprüche, **dadurch gekennzeichnet, dass** das Implantatsystem (1) eine Steuereinheit (9) umfasst, mittels welcher eine Stromstärke zwischen den Elektroden (2, 3) oder ein Potential der Arbeitselektrode (2) regulierbar ist.

8. Implantatsystem nach einem der vorherigen Ansprüche, **dadurch gekennzeichnet, dass** die Steuereinheit (9) einen Potentiostaten (10) beinhaltet.

9. Implantatsystem nach einem der vorherigen Ansprüche, **dadurch gekennzeichnet, dass** die Spannungs- oder Stromquelle (4) implantierbar und in vivo von den Elektroden (2, 3) trennbar ist.

10. Implantatsystem nach einem der vorherigen Ansprüche, **dadurch gekennzeichnet, dass** das Implantat (2) die Anode der elektrochemischen Zelle bildet.

11. Implantatsystem nach einem der vorherigen Ansprüche, **dadurch gekennzeichnet, dass** das Implantat (2) die Kathode der elektrochemischen Zelle bildet.

12. Implantatsystem nach einem der vorherigen Ansprüche, **dadurch gekennzeichnet, dass** die Elektrode(n) (2, 3) implantierbar und innerhalb des Körpergewebes (5) mit vorzugsweise biodegradierbaren Leitungen (11) versehen sind.

13. Verfahren zur Beeinflussung der Korrosionsrate von temporären Implantaten (2) aus einem metallischen, biokompatiblen und biokorrodierbaren Werkstoff, **dadurch gekennzeichnet, dass** das Implantat (2) als eine erste Arbeitselektrode (2) ausgebildet und dem Patienten implantiert wird und dass die Arbeitselektrode (2) elektrisch leitend sowie über Körpergewebe (5) des Patienten als Elektrolyt mit einer Gegenelektrode (3) verbunden wird, wobei eine elektrochemische Zelle gebildet und die Korrosionsrate des Implantats (2) in vivo gesteuert wird.

14. Verfahren nach dem vorherigen Anspruch, **dadurch gekennzeichnet, dass** das Implantat (2) und die Gegenelektrode (3) mit einer Spannungs- oder Stromquelle (4) verbunden werden.

15. Verfahren nach dem vorherigen Anspruch, **dadurch gekennzeichnet, dass** die Gegenelektrode (3) und/oder die Spannungs- oder Stromquelle (4) zusammen mit dem Implantat (2) implantiert werden.

16. Verfahren nach einem der vorherigen Ansprüche, **dadurch gekennzeichnet, dass** dem Implantat (2) eine externe Referenzelektrode (6) zugeordnet wird, welche über eine Brücke (8) mit dem Körpergewebe (5) verbunden wird oder direkt implantiert wird.

17. Verfahren nach einem der vorherigen Ansprüche, **dadurch gekennzeichnet, dass** die Elektrode(n) (2', 3) und/oder die Brücke (8) nach Implantation der Arbeitselektrode (2) perkutan in das Körpergewebe (5) eingebracht wird.

18. Verfahren nach einem der vorherigen Ansprüche, **dadurch gekennzeichnet, dass** die Arbeitselektrode (2) anodisch polarisiert wird.

19. Verfahren nach einem der vorherigen Ansprüche, **dadurch gekennzeichnet, dass** die Arbeitselektrode (2) kathodisch polarisiert wird.

20. Verfahren nach einem der vorherigen Ansprüche, **dadurch gekennzeichnet, dass** die Polarisierung der Arbeitselektrode (2) potentiostatisch reguliert wird.

21. Verfahren nach einem der vorherigen Ansprüche, **dadurch gekennzeichnet, dass** die Polarisierung der Arbeitselektrode (2) galvanostatisch reguliert wird.

22. Verfahren nach einem der vorherigen Ansprüche, **dadurch gekennzeichnet, dass** die Polarisierung der Arbeitselektrode (2) in Abhängigkeit eines Korrosionszustandes des Implantats (2) in vivo und/oder eines Krankheits-/Genesungsbildes reguliert wird.
